(19)

Europäisches
Patentamt
European
Patent Office
Office européen
des brevets

(11) **EP 4 628 074 A1**

(12) **EUROPEAN PATENT APPLICATION**

(43) Date of publication:
08.10.2025 Bulletin 2025/41

(21) Application number: 25156611.3

(22) Date of filing: 07.02.2025

(51) International Patent Classification (IPC):
*A61K 31/164* (2006.01)    *A61K 31/196* (2006.01)
*A61K 9/00* (2006.01)    *A61K 9/107* (2006.01)
*A61K 9/48* (2006.01)    *A61K 31/5415* (2006.01)
*A61P 29/00* (2006.01)    *A61K 31/192* (2006.01)

(52) Cooperative Patent Classification (CPC):
(C-Sets available)
A61K 31/196; A61K 9/00; A61K 9/107; A61K 9/48;
A61K 31/164; A61K 31/192; A61K 31/5415;
A61P 29/00                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.02.2024   IT 202400003187**

(71) Applicant: **EPITECH GROUP S.p.A.
20144 Milano (MI) (IT)**

(72) Inventors:
• **DELLA VALLE, Maria Federica**
  I-20144 Milano (IT)
• **DELLA VALLE, Raffaella**
  I-20144 Milano (IT)
• **MARCOLONGO, Gabriele**
  I-20144 Milano (IT)
• **GHELARDINI, Carla**
  I-20144 Milano (IT)
• **DI CESARE MANNELLI, Lorenzo**
  I-20144 Milano (IT)
• **CUZZOCREA, Salvatore**
  I-20144 Milano (IT)
• **ZUSSO, Morena**
  I-20144 Milano (IT)

(74) Representative: **Long, Giorgio
Jacobacci & Partners S.p.A.
Via Senato, 8
20121 Milano (IT)**

Remarks:
Claims 16 to 20 are deemed to be abandoned due to non-payment of the claims fees (Rule 45(3) EPC).

(54) **N-PALMITOYLETHANOLAMIDE FOR USE IN COMBINATION WITH A NON-STEROIDAL ANTI-INFLAMMATORY DRUG IN THE TREATMENT OF INFLAMMATORY PAIN**

(57)    The present invention relates to the use of N-palmitoylethanolamide (known as palmitoylethanolamide or PEA) in combination with a non-steroidal anti-inflammatory drug in the treatment of inflammatory pain.

In particular, the present invention is directed to palmitoylethanolamide for use in the treatment of inflammatory pain, in particular non-neuropathic inflammatory pain, where palmitoylethanolamide is administered as needed in association or combination with a non-steroidal anti-inflammatory drug, where said administration is separate, combined, or simultaneous.

**EP 4 628 074 A1**

(52) Cooperative Patent Classification (CPC): (Cont.)

C-Sets
**A61K 31/164, A61K 2300/00;**
**A61K 31/192, A61K 2300/00;**
**A61K 31/196, A61K 2300/00;**
**A61K 31/5415, A61K 2300/00**

**Description**

Technical field of the invention

The present invention relates to the use of N-palmitoylethanolamide (known as palmitoylethanolamide or PEA) in combination with a non-steroidal anti-inflammatory drug in the treatment of inflammatory pain.

Background art

Pain is one of the most common human health problems. Moreover, in recent years there has been growing attention to the recognition and management of pain also in pets, and in particular cats and dogs. In fact, based on the latest definition of pain issued by the *International Association for the Study of Pain* (IASP), to experience pain it is not necessary to know how to express it verbally, which fully includes even non-verbal subjects (such as animals) among beings capable of experiencing pain.

Based on duration, pain is classified as acute, persistent or chronic; based on origin, it is defined as nociceptive, inflammatory, or neuropathic. In conditions of tissue damage, inflammation is responsible for the production and release of mediators involved in the enhancement of pain, which in this case is defined as inflammatory.

Arachidonic acid derivatives cover an important role among these mediators: in fact, prostaglandins, prostacyclins and thromboxanes (prostanoids), produced by the action of the enzymes cyclooxygenase (COX)-1 (constitutive isoform) and COX-2 (inducible isoform), are involved in the hyper-excitation of nociceptors resulting in the appearance of allodynia.

Non-steroidal anti-inflammatory drugs or NSAIDs are the most widely used medicines in treating inflammatory pain, by virtue of the inhibitory mechanism thereof on COX. Diclofenac and meloxicam are two of the most common NSAIDs used for treating inflammatory pain, both in the human and veterinary context.

Diclofenac, an acetic acid derivative, is used in treating inflammatory and degenerative rheumatic conditions, in diseases characterized by pain and inflammation in the tissues surrounding joints such as tendons and ligaments, in painful post-traumatic states and in menstrual pain. It is available in several formulations (e.g., oral, parenteral, rectal) and different dosages depending on the therapeutic indication and severity of symptoms. In any case, it is recommended not to exceed 150 mg per day and to use the product for a limited time.

Meloxicam has a relative selectivity of action on COX-2 and belongs to that subgroup of NSAIDs called "non-coxib COX-2 selective NSAIDS". It is used for the short-term treatment of osteoarthrosis exacerbations and in the long-term treatment of pain associated with rheumatoid arthritis and ankylosing spondylitis symptoms. It is marketed in the form of different pharmaceutical formulations and it is recommended not to exceed 15 mg per day. It is also widely used in veterinary medicine, especially in treating arthrosis and post-operative pain in cats and dogs.

Although widely used in the management of inflammation and pain, NSAIDs exert serious side effects which, even today, are a cause for concern (Samal et al., J Maxillofac Oral Surg. 2021 Mar;20(1):63-69; Ringsten et al., Cochrane Database Syst Rev. 2023 Dec 11;12(12):CD015087 ; Bindu et al., Biochem Pharmacol. 2020 Oct;180:114147; Lascelles et al., Vet Ther. 2005 Fall;6(3):237-51; Wernham et al., Aust Vet J. 2023 Mar;101(3):90-98). In fact, the inhibition of prostaglandin production leads to the development of adverse events of a dose-dependent nature, with renal and hepatic toxicity, cardiovascular events, hypertension and gastrointestinal complications, especially in frail patients such as the elderly, renal patients or those undergoing multiple therapies.

The need is therefore felt to have effective and safe therapies for the correct management of inflammatory pain, both acute and chronic. To this end, it is desirable to be able to reduce the effective dosage of NSAIDs.

N-palmitoylethanolamide (or simply palmitoylethanolamide or PEA), a palmitic acid amide normally found in animal tissues, where it is produced on demand under damaging conditions, is known to carry out anti-inflammatory and anti-nociceptive actions. Preclinical and clinical studies have demonstrated the efficacy of the administration of PEA, especially in micronized form (particle size between 0.2 and 10 $\mu$M), in different types of inflammation and pain.

The analgesic effect thereof has also been compared with that of NSAIDs, and more in particular ibuprofen and celecoxib, in patients with temporomandibular pain and chronic pelvic pain. Furthermore, one study demonstrated the effect of continuous administration for 2 weeks of a combination of PEA and celecoxib on temporomandibular pain, without, however, comparing the effect with single treatments. It should be taken into account that PEA has an excellent safety profile and is free of acute and sub-chronic toxicity at least up to 1000 mg/kg daily (when administered in ultra-micronized form, Nestmann Food Sci Nutr. 2016 Jun 15;5(2):292-309).

The joint use of PEA and paracetamol, an antipyretic and analgesic drug which does not belong to the NSAID category and acts by means of mainly central mechanisms, was investigated, highlighting the benefit thereof under conditions of neuropathic pain, both experimentally caused and spontaneous. This is probably due to the fact that PEA, in particular in ultra-micronized form, also has a strong anti-inflammatory effect at the level of the central nervous system.

However, it is important to emphasize that the therapeutic improvement due to the combined use of different analgesic drugs is not obvious, as demonstrated by the fact that the association between NSAIDs and paracetamol does not entail any additional benefit.

Continuous and prolonged prophylactic use (three months) of ultra-micronized PEA, combined with the administration of one or more NSAIDs (ibuprofen, diclofenac or nimesulide) on demand was advantageous with respect to the use on demand of NSAIDs alone, in patients with migraine with and without aura. The advantage occurred from the second or third month of treatment with PEA and affected patients with migraine, considered a "syndrome" associated with central alterations typical of neuropathic pain. However, the existence of synergistic interactions between PEA and NSAIDs has not been demonstrated.

In conclusion, the prior art has not described or suggested the treatment as needed with PEA in association with an NSAID, in particular in non-neuropathic inflammatory pain conditions.

Summary of the invention

The present invention derives from the surprising finding that palmitoylethanolamide (PEA), preferably if used in ultra-micronized form, when administered in combination with a non-steroidal anti-inflammatory drug, exhibits a synergistically relevant effect in treating inflammatory pain, in particular non-neuropathic inflammatory pain, resulting in a benefit for patient safety, in terms of lower incidence and severity of dose-dependent side effects, typical of these drugs. In particular, the synergistic effect between PEA and NSAIDs in treating inflammatory pain in humans and animals allows enhancing the anti-inflammatory effects of NSAIDs, reducing the active dosage thereof.

Therefore, the present invention relates to palmitoylethanolamide for use in the treatment of inflammatory pain, in particular non-neuropathic inflammatory pain, where palmitoylethanolamide is administered as needed in association or combination with a non-steroidal anti-inflammatory drug, where said administration is separate, combined, or simultaneous.

The invention further relates to a composition containing palmitoylethanolamide and a non-steroidal anti-inflammatory drug, in particular when usable in the treatment of inflammatory pain.

These and further objects, as outlined in the appended claims, will be described in the following description. The text of the claims should be considered included in the description in order to assess the sufficiency of description.

Further features and advantages of the invention will become apparent from the following description of preferred embodiments, given by way of non-limiting indication.

Brief description of the drawings

Figure 1 shows the inflammatory pain tolerance threshold in response to the various treatment groups indicated in the legend, measured in the paw pressure test;

Figure 2 shows the comparison between the mean values of AUC $\pm$ SEM, obtained starting from the data shown in Figure 1;

Figure 3 shows the inflammatory pain tolerance threshold in response to the various treatment groups indicated in the legend, measured in the paw pressure test;

Figure 4 shows the comparison between the mean values of AUC $\pm$ SEM, obtained starting from the data shown in Figure 3;

Figure 5 shows a particle size distribution curve of ultra-micronized PEA according to an embodiment, obtained by the laser scattering method described below;

Figure 6 shows the effect of treatments on the severity of arthritis starting from the first day of treatment (day 25) and until the end of the study; the score is expressed as mean $\pm$ SEM. *$p < 0.05$ and ** $p < 0.0001$ vs. vehicle; # $p < 0.0001$ vs. IBUld; § $p < 0.05$ and §§ $p < 0.0001$ vs PEA;

Figure 7 shows the effect of treatments on paw swelling; the increase in paw volume is expressed as mean $\pm$ SEM. *$p < 0.05$ and ** $p < 0.0001$ vs vehicle; # $p < 0.0001$ vs. IBUld; § $p < 0.05$ and §§ $p < 0.0001$ vs PEA;

Figure 8 shows the effect of treatments on weight loss induced by arthritis pain; the arrow indicates the start of treatment; values are expressed as mean $\pm$ SEM. *$p < 0.05$ and ** $p < 0.0001$ vs vehicle; # $p < 0.0001$ vs IBUld; § $p < 0.05$ and §§ $p < 0.0001$ vs PEA;

Figure 9 shows the analgesic effect of the treatments; the effect is measured using Von Frey filaments and expressed as a pain tolerance threshold in grams. * = $p < 0.05$ vs veh; # = $p < 0.05$ vs IBU-ld; § = $p < 0.05$ vs PEA;

Figure 10 shows the effect of chronic treatment (14 days) with the tested substances, used individually or in combination, on the nociceptive threshold measured with the paw pressure test; the significant reduction in the threshold induced by CFA is effectively counteracted by all the treatments tested; *$p < 0.05$ and **$p < 0.0001$ vs veh; # $p < 0.05$ and ## $p < 0.0001$ vs ctrl; § $p < 0.05$ and §§ $p < 0.0001$ vs Decl.

Detailed description of the invention

In a first aspect, the present invention relates to palmitoylethanolamide for use in the treatment of inflammatory pain, in particular non-neuropathic inflammatory pain, where palmitoylethanolamide is administered as needed in association or combination with a non-steroidal anti-inflammatory drug, where said administration is separate, combined, or simultaneous.

The terms "in association" or "in combination" mean both a combination therapy and a therapy in which PEA and the non-steroidal anti-inflammatory drug (hereinafter referred to as NSAIDs) are contained in a single dosage form.

The term "as needed" means an administration, also known as "on demand", which can include a single dose or several doses and which can comprise a single administration or several administrations within a time interval between a day and a week and which includes the administration of PEA and NSAID after the onset of non-neuropathic inflammatory pain. Such a term must be understood to exclude continuous preventive, prophylactic, or curative administration.

The term "continuous administration" means an administration of several doses of the drug for a time greater than one week, more typically greater than one month.

"Separate" administration means an administration of PEA and NSAID, administered at different times ranging from 1 minute to several hours, for example 8, 12 or 14 hours apart.

"Combined" administration means an administration of PEA and NSAID contained in a single dosage form, i.e., a pharmaceutical or veterinary composition or formulation.

"Simultaneous" administration means an administration of PEA and NSAID in separate dosage forms, but administered simultaneously, i.e., within a separation time between the PEA and NSAID administration, or vice versa, not exceeding 1 minute.

Palmitoylethanolamide can be administered in any form, for example in a non-micronized form, in a micronized form or in an ultra-micronized form.

The term "palmitoylethanolamide (or PEA) in a non-micronized form" means PEA having a particle size distribution, defined as a percentage by volume and measured with the laser light scattering method, represented by a distribution curve having the mode above 10 microns, preferably above 20 microns.

The term "palmitoylethanolamide (or PEA) in a micronized form" means PEA having a particle size distribution, defined as a percentage by volume and measured with the laser light scattering method, represented by a distribution curve having the mode between 6 microns and 10 microns.

The term "palmitoylethanolamide (or PEA) in an ultra-micronized form" means PEA having a particle size distribution, defined as a percentage by volume and measured with the laser light scattering method, represented by a distribution curve having the mode below 6 microns and above 0.5 microns.

Preferably, PEA is in an ultra-micronized form.

In an embodiment, PEA in an ultra-micronized form has a particle size distribution as defined above, measured with a Malvern Mastersizer 3000 instrument with Fraunhofer calculation algorithm, where at least 90% by volume, more preferably at least 95% by volume, of particles has a particle size of less than 6 microns (d90= 6 micron).

In a particularly preferred embodiment, PEA in an ultra-micronized form has a particle size distribution as defined above, measured with a Malvern Mastersizer 3000 instrument with Fraunhofer calculation algorithm, having a mode between 2 and 4 microns and having 100% by volume of particles smaller than 10 microns and at least 60% by volume of particles smaller than 3 microns.

It must be considered that, as defined in the European Pharmacopoeia (section 2.9.31), the particle size measurements carried out with the laser light diffraction method and expressed as d90 (maximum size of 90% by volume of the particles present in a sample) have a variability of $\pm 15\%$ for d90 greater than 10 microns and $\pm 30\%$ for d90 less than 10 microns. This means that a d90 of 6 microns measured with said method is to be understood in practice as being contained in a range between 4.2 and 7.8 microns. In other words and by way of example, a d90= 7 microns measured by laser light diffraction on a sample falls within the definition of d90= 6 microns as defined in the present patent application.

The micronization can be carried out in a fluid jet system (for example, Jetmill® model system) which operates with spiral technology with a compressed air or nitrogen jet capable of exploiting kinetic energy - instead of mechanical energy - to crush the particles. Such apparatuses are conventional and will therefore not be further described, except in relation to the following features:

- Internal diameter of the micronization chamber about 300 mm;
- Fluid jet pressure 10-12 bar;
- Product supply 9-12 kg/h.

The present invention further relates to a composition comprising palmitoylethanolamide and an NSAID. Preferably, the composition of the invention consists of a dry mixture of palmitoylethanolamide/NSAID. More preferably, palmitoylethanolamide is in micronized (m-PEA) or ultra-micronized (um-PEA) form, even more preferably palmitoylethanolamide is um-PEA, or is a mixture of at least two choices from um-PEA and/or m-PEA and/or non-micronized PEA.

NSAID is preferably selected from:

- Salicylates, e.g., acetylsalicylic acid
- Acetic acid derivatives and the like, e.g., indomethacin, diclofenac, ketorolac, aceclofenac
- Propionic acid derivatives, e.g., ibuprofen, ketoprofen and naproxen
- Oxicam derivatives, e.g., piroxicam and meloxicam
- Fenamates, e.g., mefenamic acid
- Coxib or COX-2 inhibitors, e.g., celocoxib, etoricoxib and parecoxib
- Nimesulide
- Morniflumate/niflumic acid.

Preferably, NSAID is selected from diclofenac, meloxicam, ibuprofen, and ketoprofen.

Whether administered separately or combined in a single formulation, PEA and NSAID are administered in a PEA/NSAID weight ratio between 20:1 and 1:1, preferably between 12:1 and 5:1. More in particular, when PEA is in ultra-micronized form, the PEA/NSAID weight ratio will preferably be between 11:1 and 3:1, more preferably between 10:1 and 5:1. When PEA is in micronized or non-micronized form, the PEA/NSAID weight ratio will preferably be between 20:1 and 5:1, more preferably between 18:1 and 10:1.

Based on such weight ratios, for which a significant synergistic effect has been highlighted, the minimum daily dose of PEA, both in a combination therapy and in a PEA/NSAID composition, will be at least between 2.5 mg/day and 120 mg/day. Preferably, in the case of using um-PEA, the minimum daily dose of um-PEA will be between 4 mg/day and 66 mg/day, while in the case of using non-micronized PEA or m-PEA the minimum daily dose will be between 5 mg/day and 120 mg/day.

Such doses may vary depending on the subject and in particular if the subject is of child, adult or elderly age.

Taking into account that, as widely known in the literature, PEA has a low toxicity, it will be possible to use higher doses of PEA than those described above, which were sufficient to obtain a synergistic effect on non-neuropathic inflammatory pain.

The additional PEA with respect to the amount of synergistic PEA with NSAID can also be present in a different form than that used in association with NSAID. For example, if PEA is in the form of um-PEA, the additional PEA may be either um-PEA, m-PEA or non-micronized PEA, or vice versa.

Therefore, the overall daily dose of PEA administered to a subject, either in the form of combination therapy or in the aforesaid composition with an NSAID, can be between 200 and 2000 mg/day, preferably between 300 and 1500 mg/day or between 400 and 1200 mg/day.

Such daily doses can be divided into dose units for an administration, for example, from 1 to 4 times a day. The dose will also depend on the route chosen for administration. It should be considered that dosage variations could be necessary depending on the patient age and weight and also on the extent of inflammatory pain to be treated. The exact dose and route of administration will ultimately be at the discretion of the attending physician.

For the purposes of the invention, PEA alone, NSAID alone or the composition containing PEA and NSAID can be included in pharmaceutical or veterinary formulations and can be formulated in dosage forms for oral, buccal, parenteral, rectal, topical, or transdermal administration.

For oral administration, the compounds of the invention can be found, for example, in the form of tablets or capsules, hard or soft, prepared in the conventional fashion with pharmaceutically acceptable excipients such as binders (e.g., pregelatinized cornstarch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or inhibiting agents (e.g., sodium lauryl sulfate). The tablets can be coated by methods well known in the art. The liquid preparations for oral administration can be, for example, in the form of solutions, syrups or suspensions or they can be freeze-dried or granulated products to be reconstituted, before use, with water or other suitable vehicles. Such liquid preparations can be prepared through conventional methods with pharmaceutically acceptable additives such as suspending agents (e.g., sorbitol syrup, cellulose derivatives, or edible hydrogenated fats); emulsifiers (e.g., lecithin or acacia); non-aqueous vehicles (e.g., almond oil, oily esters, ethyl alcohol or fractionated vegetable oils); and preservatives (e.g., methyl- or propyl-p-hydroxybenzoates, sorbic acid, benzoic acid or salts thereof). The preparation can also conveniently contain flavorings, dyes, and sweeteners.

The preparations for oral administration can be appropriately formulated to allow the controlled release of the active constituent.

For buccal administration, the compounds of the invention can be in the form of tablets or granules formulated in the conventional manner, which are suitable for an absorption at the level of the buccal mucosa. Typical buccal formulations are tablets for sublingual administration.

The compounds of the invention can be formulated for parenteral administration by injection. The injection formulations can be presented as a single dose, for example in vials, with an added preservative. The compositions can appear in such a form as suspensions, solutions, or emulsions in oily or aqueous vehicles and can contain agents of the formulation such as suspension, stabilizers and/or dispersants. Alternatively, the active ingredient or the mixture of active ingredients can be found in the form of a powder to be reconstituted, before use, with a suitable vehicle, for example with sterile water.

The compounds of the invention can also be formulated according to rectal formulations such as suppositories or retention enemas, for example containing the basic components of the common suppositories such as cocoa butter or other glycerides.

In addition to the formulations described above, the compounds of the invention can also be formulated as a deposit preparation for administration over a day to a week. Such long-acting formulations can be administered by implantation (e.g., subcutaneously, transcutaneously or intramuscularly) or intramuscular injection. Therefore, for example, the composition can be formulated with appropriate polymer or hydrophobic materials (for example in the form of an emulsion in a suitable oil) or ion exchange resins or as minimally soluble derivatives.

The compounds or composition of the invention can also be administered in the form of oral sprays or nasal sprays.

The invention further relates to a composition comprising or consisting of a mixture of palmitoylethanolamide, preferably ultra-micronized palmitoylethanolamide, a non-steroidal anti-inflammatory drug and pharmaceutically acceptable excipients, where the PEA/NSAID weight ratio is between 20:1 and 1:1, preferably between 12:1 and 5:1, where PEA is contained in an amount between 200 and 2000 mg and where NSAID is preferably selected from diclofenac, meloxicam, ibuprofen, and ketoprofen.

In certain embodiments, the aforesaid composition, or palmitoylethanolamide mixture for separate or sequential administration, further comprises 2-pentadecyl-2-oxazoline (also referred to as PEA-OXA) which is a PEA analog.

A further object of the invention is 2-pentadecyl-2-oxazoline for use in the treatment of inflammatory pain, in particular non-neuropathic inflammatory pain, in which 2-pentadecyl-2-oxazoline is administered in association or combination with a non-steroidal anti-inflammatory drug, in which said administration is separate, joint or simultaneous and in which, preferably, the non-steroidal anti-inflammatory drug is diclofenac.

The weight ratio 2-pentadecyl-2-oxazoline/diclofenac is preferably 5:3 or greater.

2-Pentadecyl-2-oxazoline is preferably administered at a dose between 100 mg and 1000 mg per day.

A further object of the invention is a composition comprising or consisting of a mixture of 2-pentadecyl-2-oxazoline, a non-steroidal anti-inflammatory drug, preferably diclofenac, and pharmaceutically acceptable excipients, in which the 2-pentadecyl-2-oxazoline is preferably contained in an amount of between 100 mg and 1000 mg and in which the weight ratio 2-pentadecyl-2-oxazoline/diclofenac is preferably 5:3 or greater.

The invention further relates to dietary compositions, food supplements, complementary feed and foods for special medical purposes (FSMPs) comprising PEA, preferably ultra-micronized PEA.

The term "foods for special medical purposes" means products authorized according to (EU) regulation 2016/128. Such a term refers to a product to be administered under medical supervision, thus assimilating such an FSMP to a drug.

The formulations according to the invention can be prepared according to conventional methods, such as those described in Remington's Pharmaceutical Sciences Handbook, Mack Pub. Co., N.Y., USA, 17th edition, 1985 o in Remington, The Science and Practice of Pharmacy, Edited by Allen, Loyd V., Jr, 22nd edition, 2012 or in subsequent editions.

EXPERIMENTAL SECTION

Micronization procedure

PEA was micronized as previously described.

The ultra-micronization was carried out in a fluid jet system (in particular, the Jetmill® model system) which operates with compressed air jet "spiral technology".

Optimal micronization conditions:

- internal diameter of the micronization chamber 300 mm;
- fluid jet pressure 8 bar;
- product supply 9-12 kg/h.

Determination of the particle size distribution

The determination of the particle size distribution was carried out on a wet sample, after 1-minute sonication.

A Malvern Mastersizer 3000 instrument operating with the LALLS (Low Angle Laser Light Scattering) technique and a Fraunhofer calculation algorithm was used.

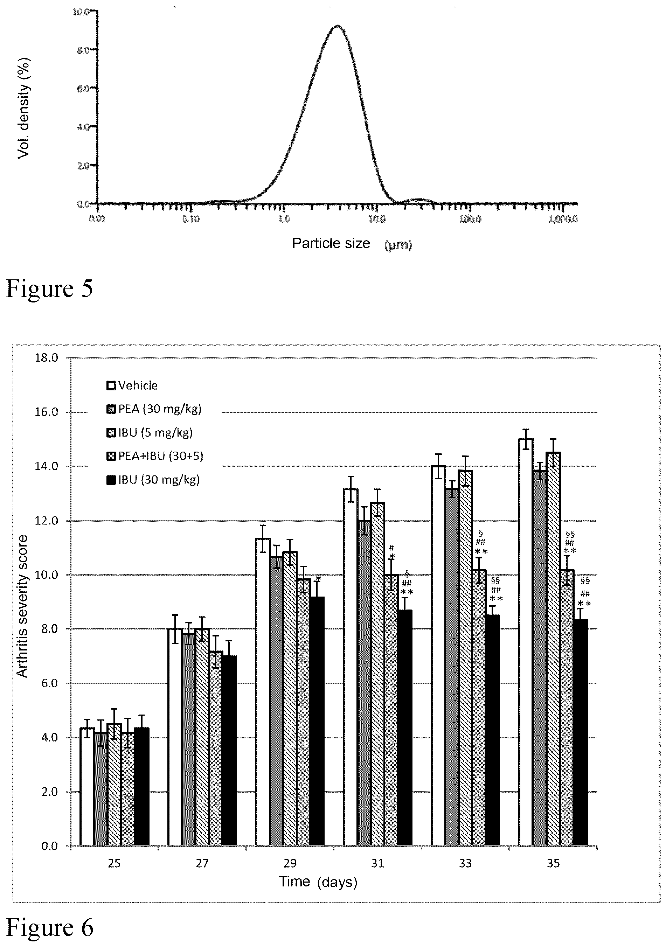

The particle size distribution graph is shown in Figure 5.

**Biological experimentation**

EXPERIMENT 1 - Efficacy and synergy of PEA and an NSAID in an animal model of CFA-induced inflammatory pain

In the *in vivo* experimentation, male Sprague-Dawley rats (200-250 g) (Envigo, Varese, Italy) fed "ad libitum" and housed in cages with a controlled sleep/wake cycle were used.

Before the start of the study, the animals were subjected to a 1-week acclimatization period at the Center for Laboratory Animal Sheltering (Ce.S.A.L.) of the University of Florence, following all experimental procedures and protocols compliant with the principles of care and welfare of laboratory animals approved by the Italian Ministry of Health (Italian Legislative Decree 2014/26), European directives (EU Directive 2010/63) and the ARRIVE guidelines.

In order to cause inflammatory pain, Complete Freund's Adjuvant (CFA) was injected at a rate of 50 μL into the joint cavity of the left leg between the tibiofibular and tarsal bones after light anesthesia with 2% isoflurane. The control group was subjected to the same procedure, injecting an equal volume of saline solution (vehicle).

The animals were divided into 9 groups of 6 rats each and treated acutely *per* os with a single administration, starting from the seventh day after the induction of joint damage.

- Group 1: healthy rats subjected to intra-articular injection of saline (vehicle group)
- Group 2: rats injected with CFA and treated with 1% CMC, corresponding to the vehicle in which the compounds for the treatments of the following groups were suspended (CFA group);
- Group 3: rats injected with CFA and treated with micronized PEA 10 mg/kg;
- Group 4: rats injected with CFA and treated with diclofenac 30 mg/kg;
- Group 5: rats injected with CFA and treated with diclofenac 3 mg/kg;
- Group 6: rats injected with CFA and treated with diclofenac 3 mg/kg + micronized PEA 10 mg/kg;
- Group 7: rats injected with CFA and treated with meloxicam 30 mg/kg;
- Group 8: rats injected with CFA and treated with meloxicam 3 mg/kg;
- Group 9: rats injected with CFA and treated with meloxicam 3 mg/kg + micronized PEA 10 mg/kg.

The micronized PEA used in the experiments (hereinafter referred to as PEA for simplicity) had an average particle size between 0.2 and 10 μM and a d90 of about 6 microns. All animals were subjected to the paw pressure test at the level of the ipsilateral paw, performed before (T0) or after 15, 30, 45 and 60 minutes after the single administration of the treatment (T15, T30, T45 and T60, respectively). In particular, the nociceptive threshold was calculated with an analgesiometer (Ugo Basile, Varese, Italy) by applying an increasing pressure at constant speed (32 g/s) by means of a non-pointed conical support on the dorsal surface of the ipsilateral paw with respect to the injection of CFA (or vehicle). The nociceptive threshold was expressed as the force (in decagrams, dag) to which the animal reacts by retracting the paw or vocalizing (Leighton GE et al. kappa-Opioid agonists produce antinociception after i.v. and i.c.v. but not intrathecal administration in the rat. Br J Pharmacol. 1988; 93: 553-60).

**Statistical analysis**

The values emerged from the paw pressure test in the different treatment groups were compared with each other using the Generalized Linear Mixed Model (GLMM) followed by post-hoc analysis based on Tukey-Kramer correction for multiple comparisons, referring to the single treatment group, considering all the times. For the synergy analysis, the area under the curve (AUC) was considered, calculated with the trapezoid rule for the following four treatment groups:

```
CFA + vehicle

CFA + PEA 10 mg/kg

CFA + NSAID 3 mg/kg

CFA + PEA (10 mg/kg) + NSAID (3 mg/kg)
```

in order to verify whether the effect of the combined administration of PEA and NSAIDs was greater than the sum of the individual effects of the two treatments used individually.

The AUC was analyzed by a two-way factor analysis of variance (2x2 ANOVA), after plotting the mean values, according to the procedure shown by Slinker BK. The statistics of synergism. J Mol Cell Cardiol. 1998 Apr;30(4):723-31. The values were expressed as mean $\pm$ standard error mean (SEM). All the statistical analyses were carried out using the software SAS, version 9.4 (SAS Institute, Cary, NC, USA). The value $p < 0.05$ was considered significant.

**Results of the experiments**

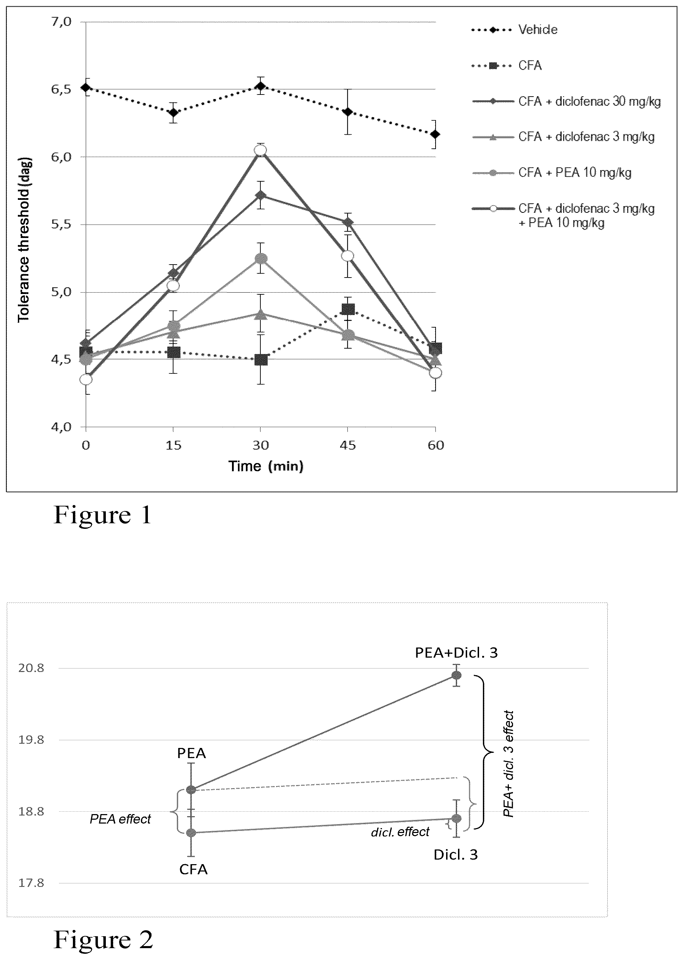

As for the experiments with diclofenac, from the comparison of the effects observed in each treatment group, it was observed that (Figure 1):

- The injection of CFA (dotted line with squares) causes significant inflammatory pain, as shown by the significant lowering of the tolerance threshold (p<0.0001) with respect to the vehicle group (dotted line with diamonds);
- Unlike the group treated with diclofenac at 3 mg/kg (line with triangles) and with PEA (line with solid circles), which show no significant difference with respect to the CFA group (p = 1.0 and p = 0.9674, respectively), the group treated with diclofenac 30 mg/kg (solid line with diamonds) shows a significant increase in the pain tolerance threshold with respect to the CFA group (p = 0.0004);
- Surprisingly, the combined use of PEA and low-dose diclofenac (3 mg/kg), which - as mentioned above - individually had no effect, significantly reduced pain (line with empty circles; p = 0.0047 with respect to CFA).

It is noteworthy that the effect produced by the aforesaid combination is not significantly different (p = 0.9890) from that obtained with diclofenac 30 mg/kg, which means that the addition of PEA to diclofenac allows obtaining the same analgesic effect for 10-fold lower dosages of NSAID.

The synergy analysis carried out on the AUC values allowed confirming a synergistic effect between PEA and low-dose diclofenac. In particular, as plotted in Figure 2, the sum of the effects exerted by the individual compounds (small curly bracket) is less than the effect exerted by the combined use of the two (large curly bracket). Graphically, this is immediately appreciable from the fact that the two segments are not parallel to each other.

The results of the ANOVA analysis are reported in Table 1, where Pr > F corresponding to the line PEA* Dicl3 represents the probability that the superiority of the effect shown by the two substances combined with respect to the sum of the effects of the individual substances is due only to chance. As can be seen, this is a probability of less than 1 in thirty, demonstrating a surprising and significant synergistic effect between PEA and low-dose diclofenac.

Table 1 - Results of the two-way factor analysis of variance (ANOVA 2x2) carried out on the data plotted in Figure 2 (PEA= um-PEA 10 mg/kg; Dicl3= diclofenac 3 mg/kg)

| Source | DF | Sum of Squares | Mean Squares | F Value | Pr > F |
|---|---|---|---|---|---|
| Dicl 3 | 1 | 5.1060 | 5.1060 | 9.99 | 0.0049 |
| PEA | 1 | 10.4544 | 10.4544 | 20.46 | 0.0002 |
| **PEA*Dicl 3** | **1** | **2.8222** | **2.8222** | **5.52** | **0.0292** |
| Error | 20 | 10.2214 | 0.51107 | | |

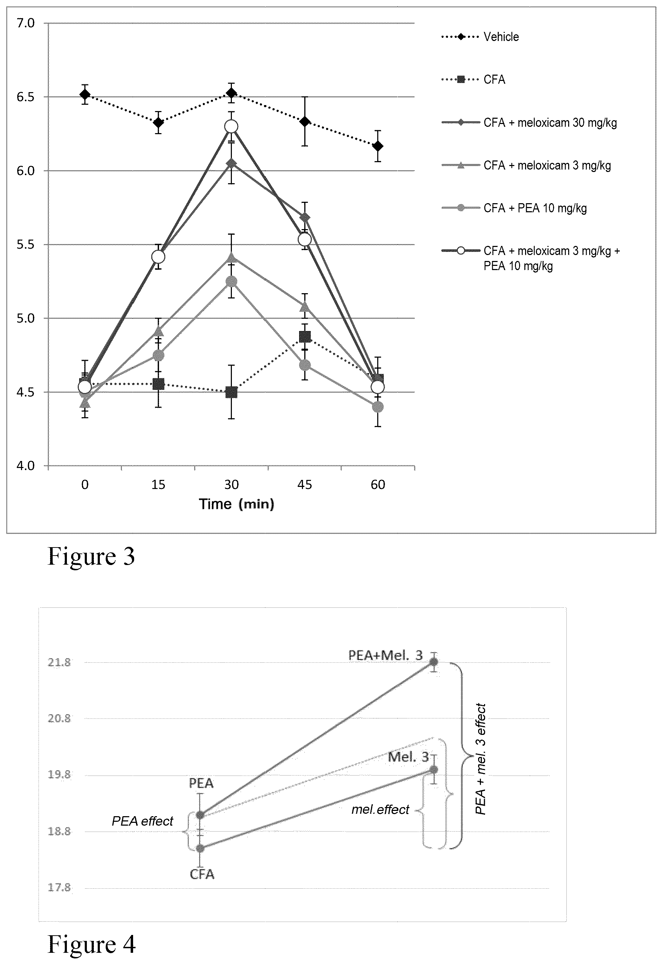

As for the experiments with meloxicam, the results obtained are completely comparable to those observed for diclofenac and described above. More specifically, it was found that (Figure 3):

- The injection of CFA (dotted line with squares) causes significant inflammatory pain, as shown by the significant lowering of the tolerance threshold (p<0.0001) with respect to the vehicle group (dotted line with diamonds);
- Unlike meloxicam at 3 mg/kg (line with triangles) and PEA alone (line with solid circles), which show no significant effect with respect to CFA (p = 0.1405 and p = 0.9588, respectively), meloxicam administered at 30 mg/kg (line with diamonds) significantly counteracts the threshold lowering caused by CFA (p = 0.0001);
- Surprisingly, the combined use of PEA and low-dose meloxicam (3 mg/kg), that - as mentioned above - individually had no effect, significantly reduces pain (line with empty circles; p < 0.0001 with respect to CFA).

Also for meloxicam, as previously detected for diclofenac, the experiments show that the effect produced by combining PEA with a low dose of meloxicam is not significantly different (p = 1.0000) from that obtained with meloxicam 30 mg/kg, which means that the addition of PEA to meloxicam allows obtaining the same analgesic effect for 10-fold lower dosages of NSAID.

The synergy analysis carried out on the AUC data allowed confirming a synergistic effect between PEA and low-dose meloxicam, both visually (Figure 4), and based on the probability which emerged from the Anova 2x2 analysis (Table 2):

Table 2 - Results of the two-way factor analysis of variance (ANOVA 2x2) carried out on the data plotted in Figure 4
(PEA= um-PEA 10 mg/kg; Mel3= meloxicam 3 mg/kg)

| Source | DF | Sum of Squares | Mean Squares | F Value | Pr > F |
|---|---|---|---|---|---|
| Mel 3 | 1 | 24.6139 | 24.6139 | 48.1 | <0.0001 |
| PEA | 1 | 9.5067 | 9.5067 | 18.58 | 0.0003 |
| **PEA*Mel 3** | **1** | **2.3406** | **2.3406** | **4.57** | **0.0450** |
| Error | 20 | 10.2342 | 0.5117 | | |

EXPERIMENT 2 - Effectiveness and synergy of PEA and Ibuprofen in a model of inflammatory pain associated with rheumatoid arthritis (CIA, collagen-induced arthritis)

Rheumatoid arthritis is a disease characterized by inflammatory pain, in which the involved joints present swelling, stiffness and often erosive processes. Female Lewis rats were maintained on a 12/12 hour light/dark cycle, at constant temperature and humidity and acclimated for 1 week, with free access to water and standard diet. Collagen induction of arthritis was performed as described in Impellizzeri D et al. J Pharmacol Exp Ther 2011, 33:859-869. Briefly, chicken collagen type II (CII) was dissolved in 0.01 M acetic acid at a concentration of 2 mg/mL, while complete Freund's adjuvant (CFA) was prepared with the addition of Mycobacterium tuberculosis H37Ra at a concentration of 2 mg/mL. The immunization emulsion was prepared by emulsifying CII in equal volume of CFA. Animals were immunized by injecting this emulsion intradermally at the base of the tail (day 0), then boosted on day 21 via a second injection. From day 25 to day 35, the animals were treated orally based on the group to which they were assigned, as outlined below (N=6 rats/group):

Sham (healthy, non-immunized and untreated control group)
Vehicle (group with CIA, treated with vehicle only)
PEA (CIA group, treated with micronized PEA at a dose of 30 mg/kg)
IBU-ld (CIA group, treated with low-dose ibuprofen, 5 mg/kg)
IBU-hd (CIA group, treated with high-dose ibuprofen, 30 mg/kg)
PEA+IBU (CIA group, treated with the combination of micronized PEA at a dose of 30 mg/kg and low-dose ibuprofen, 5 mg/kg).

Arthritis score, change in paw volume (mL), and change in body weight (g) were measured as indicators of inflammatory pain. Arthritis severity was measured every other day starting on day 25, using the following score: 0 = no sign of arthritis; 1 = swelling and/or redness of the paw or toe; 2 = two joints involved; 3 = more than two joints involved; and 4 = severe arthritis of the entire paw and toes. The arthritis index for each subject was calculated by adding the four scores of the individual paws. The change in paw volume compared to day 21 was assessed every two days by plethysmometry (Ugo Basile) starting from day 25. The change in weight was measured for each animal on day 21 and then every two days starting from day 25.

### Statistical analysis

The chosen inflammatory pain indicators were analyzed via Generalized Linear Model (GLM) followed by post-hoc analysis based on Tukey-Kramer correction for multiple comparisons. In order to verify whether the association of PEA and IBU exerted a synergistic effect (i.e., produced an effect greater than the sum of the effects of the two treatments used individually), factorial analysis of variance (ANOVA 2x2) was used, according to the procedure illustrated by Slinker BK (1998). The results of the analyzes were expressed as mean $\pm$ standard error of the mean (SEM). All analyzes were performed using SAS software, version 9.4 (SAS Institute, Cary, NC, USA). P value < 0.05 was considered significant.

### Results of the experiments

After six days of treatment (i.e., starting from day 31) the first significant effects were evident. More specifically, the score recorded in the PEA+IBU group (10.0 ± 0.58) and in the IBUhd group (8.7 ± 0.49) was significantly lower than that recorded in the untreated group (vehicle, 13.2 ± 0.48; P=0.0017 and p<0.0001, respectively). The same observations emerged at subsequent times, up to the end of the study (day 35), confirming that only the association of PEA + IBU and high-dose IBU were effective in reducing the arthritis score, while PEA alone and Low-dose IBUs had no effect, as shown in figure 6.

At the end of the treatment, it emerged that PEA and low-dose ibuprofen (IBUld) exerted a synergistic effect. As reported in the last column of the table resulting from the 2x2 factorial analysis of variance (Table 3), the probability that the effect of the combination was greater than the sum of the effects of the individual substances was, in fact, less than 0.05 (p = 0.0018). Interestingly, the effect of PEA and low-dose ibuprofen was synergistic already on day 33 (p = 0.0052).

Table 3 - Synergistic effect of the association of PEA and low-dose ibuprofen on the severity of arthritis. Results of the 2x2 factor analysis

|  | | DF | Sum of Squares | Mean Squares | F Value | Pr > F |
|---|---|---|---|---|---|---|
| Giorno 33 | PEA | 1 | 30,4 | 30,4 | 24,8 | <0,0001 |
| | IBU | 1 | 15 | 15 | 12,28 | 0,0022 |
| | PEA*IBU | 1 | 12 | 12 | 9,83 | **0,0052** |
| | Error | 20 | 24,5 | 1,2 | | |
| Giorno 35 | PEA | 1 | 45,4 | 45,4 | 39,17 | <0,0001 |
| | IBU | 1 | 26 | 26 | 22,48 | 0,0001 |
| | PEA*IBU | 1 | 15 | 15 | 12,99 | **0,0018** |
| | Error | 20 | 23,2 | 1,2 | | |

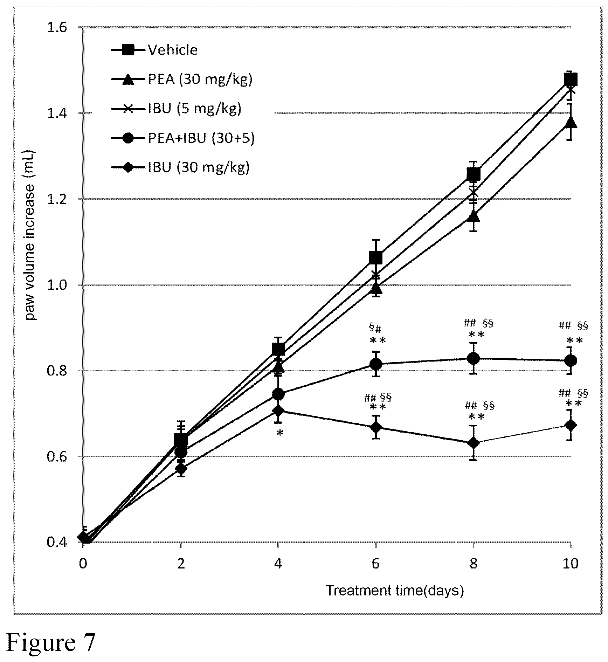

Completely similar results emerged from the plethysmometric analysis, with the onset of effects starting from the sixth day, when the association of PEA + IBU (0.82 ± 0.03 mL) and high-dose IBU (0.067 ± 0.03 mL) significantly counteracted the volume increase compared to vehicle (1.06 ± 0.04 mL; p < 0.0001 for both comparisons). On the contrary, the single administration of PEA and IBU had no effect, as shown in figure 7.

The result of the 2x2 factorial analysis demonstrated the synergistic effect of PEA and low-dose IBU, not only at the end of the treatment, but already starting from the sixth day. At these observation times, in fact, the effect obtained with the combination of PEA and low-dose ibuprofen was confirmed to be significantly higher than the sum of the effects of the individual treatments. The results of the analysis are reported in Table 4:

Table 4 - Table resulting from the 2x2 factorial analysis; the synergistic effect of the combination of PEA and low-dose ibuprofen on arthritis pain (measured as swelling of the paw) manifests itself starting from the sixth day of treatment and is demonstrated by the fact that the effect of the combined treatment (PEA*IBU) is significantly higher than the sum of the effects of the individual treatments, as highlighted in bold in the last column of the table

| 6° giorno di trattamento | | | | |
|---|---|---|---|---|
| Source | DF | Sum of Squares | Mean Squares | F Value | Pr > F |
| PEA | 1 | 0,12 | 0,12 | 19,91 | 0,0002 |
| IBU | 1 | 0,07 | 0,07 | 12,25 | 0,0023 |
| PEA*IBU | 1 | 0,03 | 0,03 | 4,92 | **0,0383** |
| Error | 20 | 0,12 | 0,01 | | |

(continued)

| 8° giorno di trattamento | | | | | |
|---|---|---|---|---|---|
| Source | DF | Sum of Squares | Mean Squares | F Value | Pr > F |
| PEA | 1 | 0,35 | 0,35 | 57,63 | <0,0001 |
| IBU | 1 | 0,21 | 0,21 | 35,00 | <0,0001 |
| PEA*IBU | 1 | 0,13 | 0,13 | 20,75 | **0,0002** |
| Error | 20 | 0,12 | 0,01 | | |

| 10° giorno di trattamento | | | | | |
|---|---|---|---|---|---|
| Source | DF | Sum of Squares | Mean Squares | F Value | Pr > F |
| PEA | 1 | 0,80 | 0,80 | 145,12 | <0,0001 |
| IBU | 1 | 0,50 | 0,50 | 91,61 | <0,0001 |
| PEA*IBU | 1 | 0,43 | 0,43 | 77,46 | **<0,0001** |
| Error | 20 | 0,11 | 0,01 | | |

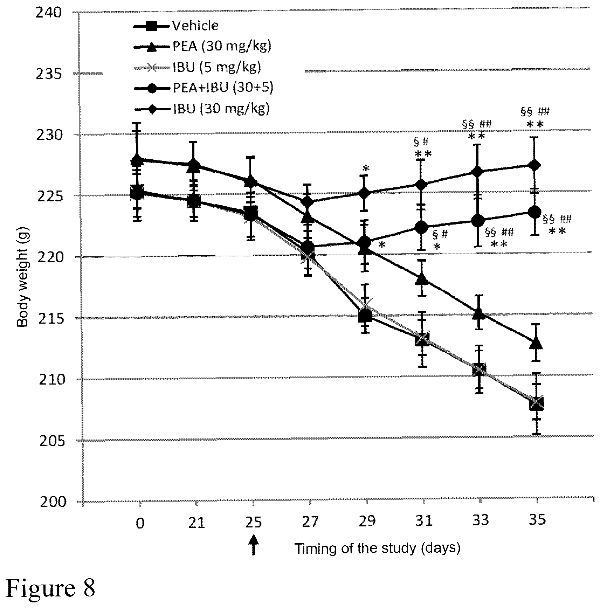

Similarly, the weight reduction induced by pain linked to the development of arthritis was significantly counteracted only by the association of PEA + IBU and high-dose IBU (Figure 8), starting from the 4th day of treatment (corresponding to day 29 of study) and for the entire observation period. When administered individually, neither PEA nor low-dose ibuprofen exerted any effect.

As in the case of the previous parameters, also for weight loss the 2x2 factorial analysis of variance highlighted a synergistic effect for the association of the two substances (PEA and low-dose Ibuprofen). The synergism, in particular, manifested itself starting from the sixth day of treatment, maintaining itself at subsequent observation times. The results of the analysis are reported in Table 5:

Table 5 - Table resulting from the 2x2 factorial analysis; the synergistic effect of the combination of PEA and low-dose ibuprofen on pain (measured as weight change) manifests itself starting from the sixth day of treatment and is demonstrated by the fact that the effect of the combined treatment (PEA*IBU) is significantly higher than the sum of the effects of the individual treatments, as highlighted in bold in the last column of the table

| 6° giorno di trattamento (G31) | | | | | |
|---|---|---|---|---|---|
| Source | DF | Sum of Squares | Mean Squares | F Value | Pr > F |
| PEA | 1 | 192,7 | 192,7 | 26,15 | <0,0001 |
| IBU | 1 | 80,7 | 80,7 | 10,95 | 0,0035 |
| PEA*IBU | 1 | 66,7 | 66,7 | 9,05 | **0,0069** |
| Error | 20 | 147,3 | 7,4 | | |

| 8° giorno di trattamento (G33) | | | | | |
|---|---|---|---|---|---|
| Source | DF | Sum of Squares | Mean Squares | F Value | Pr > F |
| PEA | 1 | 301,0 | 301,0 | 33,73 | <0,0001 |
| IBU | 1 | 165,4 | 165,4 | 18,53 | 0,0003 |
| PEA*IBU | 1 | 155,0 | 155,0 | 17,37 | **0,0005** |
| Error | 20 | 178,5 | 8,9 | | |

(continued)

| 10° giorno di trattamento (G35) | | | | | |
|---|---|---|---|---|---|
| Source | DF | Sum of Squares | Mean Squares | F Value | Pr > F |
| PEA | 1 | 477,0 | 477,0 | 49,48 | <0,0001 |
| IBU | 1 | 287,0 | 287,0 | 29,77 | <0,0001 |
| PEA*IBU | 1 | 260,0 | 260,0 | 26,97 | **<0,0001** |
| Error | 20 | 192,8 | 9,6 | | |

EXPERIMENT 3 - Effectiveness and synergy of PEA and Ibuprofen in a model of inflammatory pain induced by sub-plantar injection of carrageenan

The experimental inflammatory pain model was induced through a sub-plantar injection of carrageenan (CAR) saline solution (0.1 mL of a 1% suspension of CAR in 0.85% saline). The experimentation was conducted on adult male Sprague-Dawley rats, including 6 animals for each of the following experimental groups:

Group 1: rats subjected to CAR injection and treated with 1% CMC, corresponding to the vehicle in which the compounds were suspended for the treatments referred to in the subsequent groups (vehicle group);
Group 2: rats injected with CAR and treated with PEA 100 mg/kg;
Group 3: rats injected with CAR and treated with Ibuprofen 5 mg/kg;
Group 4: rats injected with CAR and treated with PEA 100 mg/kg + Ibuprofen 5 mg/kg;
Group 5: rats injected with CAR and treated with Ibuprofen 30 mg/kg.

The animals were treated orally with a single administration, 30 minutes before the carrageenan injection. The PEA used in this experiment was in unprocessed (native) form. Von Frey filaments were used to evaluate pain, recording for each animal the mechanical tolerance threshold, i.e. the pressure (expressed in grams) endured before retracting the paw. The measurement took place using an analgesimeter (Ugo Basile, Comerio, Varese, Italy) and was conducted before the sub-plantar injection of the CAR and at the time of maximum damage (5 hours later).

## Statistical analysis

The effect on pain was analyzed using Generalized Linear Model (GLM) followed by post-hoc analysis based on Tukey-Kramer correction for multiple comparisons. In order to verify whether the association of PEA and IBU exerted a synergistic effect (i.e., produced an effect greater than the sum of the effects of the two treatments used individually), factorial analysis of variance (ANOVA 2x2) was used, according to the procedure illustrated by Slinker BK (1998). The results of the analyses were expressed as mean $\pm$ standard error of the mean (SEM). All analyses were performed using SAS software, version 9.4 (SAS Institute, Cary, NC, USA). P value < 0.05 was considered significant.

## Results of the experiments

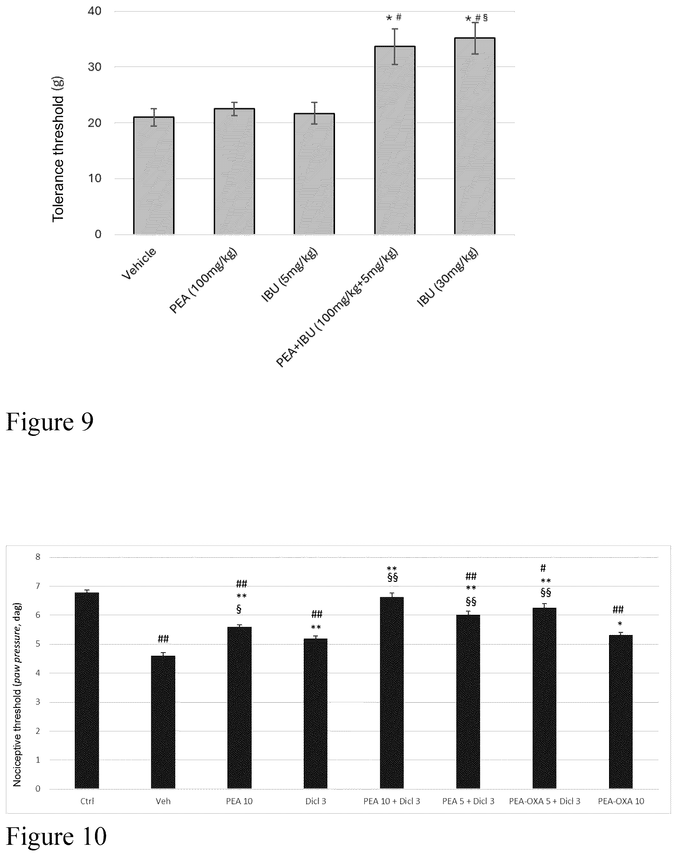

As illustrated in Figure 9, joint treatment with PEA and low-dose ibuprofen (but not single treatments) significantly raises the tolerance threshold compared to the vehicle-treated group (p = 0.0048). Surprisingly, the effect was comparable to that of high-dose ibuprofen (p = 0.9897), meaning that the addition of PEA at an inactive dose allows a significant analgesic effect to be obtained with an equally inactive dose of ibuprofen (5 mg/kg), a dose 6 times lower than that shown to be active (30 mg/kg).

The 2x2 ANOVA factorial analysis also highlighted that PEA and low-dose ibuprofen had a synergistic effect on pain, as confirmed by the fact that the effect of the association was significantly greater than the sum of the effects of the individual substances (p < 0, 05; Table 6)

Table 6 - Table resulting from the 2x2 factorial analysis; the synergistic effect of the association of PEA and low-dose ibuprofen is demonstrated by the fact that the effect of the combined treatment (PEA*Ibu) is significantly higher than the sum of the effects of the individual treatments, as highlighted in bold in the last column of the tabulated

| Source | DF | Sum of Squares | Mean Squares | F Value | Pr > F |
|---|---|---|---|---|---|
| PEA | 1 | 273,4 | 273,4 | 10,2 | 0,0046 |
| Ibuprofen | 1 | 210,0 | 210,0 | 7,83 | 0,0111 |
| PEA*Ibu | 1 | 165,4 | 165,4 | 6,17 | **0,0220** |
| Error | 20 | 536,2 | 26,8 | | |

EXPERIMENT 4 - Efficacy and synergy of the chronic administration of PEA or PEA-OXA and Diclofenac in an animal model of inflammatory pain induced by CFA

The inflammatory pain model was induced by injecting Complete Freund's Adjuvant (CFA, Sigma-Aldrich) into the tibiotarsal joint of male Sprague-Dawley rats (Charles River), housed in 26x41 cm cages at the Animal Husbandry Centre. Laboratory Animals (Ce.S.A.L.) of the University of Florence with a 12-hour circadian cycle, water and food (standard diet) "ad libitum", temperature of $23\pm1°C$. Intra-articular injection occurred according to the method described by Butler et al. (Pain 48:73-81,1992) after light anesthesia with 2% isoflurane. The skin surrounding the injection site was sterilized with 75% ethyl alcohol. Then a 28-gauge needle was inserted into the joint cavity, through which a volume of 50 $\mu$l of CFA was injected. The animals were then divided into the following treatment groups (N = 8):

Sham (control, a volume of physiological solution equal to that used for CFA animals was injected into the tibiotarsal joint)
Vehicle (CMC 1%)
Diclofenac (3 mg/kg)
Diclofenac (30 mg/kg)
PEA (10 mg/kg)
PEA-OXA (10 mg/kg)

```
PEA (5 mg/kg) + Diclofenac (3 mg/kg)

PEA (10 mg/kg) + Diclofenac (3 mg/kg)

PEA (10 mg/kg) + Diclofenac (30 mg/kg)

PEA-OXA (5 mg/kg) + Diclonfenac (3 mg/kg).
```

The tested molecules - used both individually and in combination - were suspended in 1% CMC and administered orally daily from day 1 to day 14 (chronic treatment). The PEA used in these experiments had an average particle size between 0.2 and 10 $\mu$m and a d90 of approximately 6 microns. Behavioral tests were conducted 24 hours after the last administration, in order to highlight the effect of repeated treatment on joint pain. Inflammatory pain induced by CFA injection was measured by paw pressure test. Briefly, before and sixty min after CFA injection, increasing pressure at a constant rate (32 g/s) was applied to the ipsilateral paw of the rat using a non-sharp conical support. The nociceptive threshold has been expressed as the force to which the animal reacts by retracting its paw or vocalizing (Leighton et al., Br J Pharmacol 93:553-560,1988). An analgesimeter (Ugo Basile, Varese) was used for this purpose, which allows the mechanical hyperalgesia to be quantified. Furthermore, spontaneous pain was assessed using the incapacitance test, which evaluates variations in postural balance through the "Incapacitance" device (Linton Instrumentation, UK), a scale which simultaneously, but separately, measures the force imparted by each paw (Fernandes et al., Arthritis Res Ther. 2016 Jan 11;18:7).
Briefly, rats were trained to stand on their hind legs in a box with an inclined plane (65° to the horizontal), placed above the "Incapacity" apparatus. In the absence of hind paw injury, rats will apply equal force with both hind paws, indicating postural balance; vice versa, an unequal weight distribution will indicate a decreased unilateral pain threshold.

The value of each animal is the result of the average of 3 consecutive measurements. The data are expressed in grams as the difference between the weight applied on the paw contralateral to the lesion and the weight applied on the ipsilateral one (Δ Weight). The results were expressed in terms of "painful potency", defined as percentage inhibition (mean ± SEM) of inflammatory pain induced by CFA and calculated according to the formula: 100* (treatm-VEIC)/(CTRL-VEIC)."

## Statistical analysis

The effect on pain was analyzed using Generalized Linear Model (GLM) followed by post-hoc analysis based on Tukey-Kramer correction for multiple comparisons. The results of the analyses were expressed as mean ± standard error of the mean (SEM). In order to verify whether the molecules exerted a synergistic effect, it was analyzed whether the extent of the inhibition of inflammatory pain by CFA was greater for the association of the two substances compared to the substances used individually. To this end, factorial analysis of variance (ANOVA 2x2) according to Slinker BK (1998) was used. All analyses were performed using SAS software, version 9.4 (SAS Institute, Cary, NC, USA). P value < 0.05 was considered significant.

## Results of the experiments

It is necessary to premise that the daily monitoring of the animals highlighted an important toxicity linked to prolonged treatment with Diclofenac at a dosage of 30 mg/kg, so much so that it was not possible to consider this treatment group for the analyses, due to the high mortality. Figure 10 shows the nociceptive threshold (in decagrams) measured by paw pressure test after 14 days of treatment. As can be seen, the addition of PEA or PEA-OXA to diclofenac brings a therapeutic advantage, demonstrated by the significant increase in the nociceptive threshold in the co-treated groups compared to the group treated with Diclofenac alone. Furthermore, it can be observed that the combination of PEA (10 mg/kg) with Diclofenac (3 mg/kg) is able to completely reverse the painful effect of CFA.

The 2X2 ANOVA factorial analysis highlighted that PEA exerts a synergistic effect compared to that of Diclofenac (3 mg/kg) in the control of spontaneous pain, as confirmed by the fact that combining PEA with Diclofenac (at 5 mg/kg or 10 mg/kg) produces a reduction in pain greater than the sum of the analgesic effects obtained with the individual substances (p = 0.014 for the association with PEA 10 mg/kg and p < 0.0001 for the association with PEA 5 mg/kg; Tables 7 and 8):

Table 7 - Table resulting from the 2x2 ANOVA factorial analysis; the synergistic effect exerted by PEA (10 mg/kg) compared to diclofenac is demonstrated by the fact that the effect of the combined treatment (PEA10*dicl3) is significantly higher than the sum of the effects of the individual treatments, as evidenced by the reported probability value in bold in the last column of the printout

| Source | DF | Sum of Squares | Mean Squares | F Value | Pr>F |
|---|---|---|---|---|---|
| PEA 10 | 1 | 0,58 | 0,58 | 8,03 | 0,0084 |
| diclofenac 3 | 1 | 0,89 | 0,89 | 12,2 | 0,0016 |
| PEA 10 * diclofenac 3 | 1 | 0,50 | 0,50 | 6,93 | **0,0137** |
| Error | 28 | 2,03 | 0,07 | | |

Table 8 - Table resulting from the 2X2 ANOVA factorial analysis as above

| Source | DF | Sum of Squares | Mean Squares | F Value | Pr > F |
|---|---|---|---|---|---|
| PEA 10 | 1 | 1,96 | 1,96 | 24,82 | <0,0001 |
| diclofenac 3 | 1 | 2,48 | 2,48 | 31,51 | <0,0001 |
| PEA5 * diclofenac 3 | 1 | 1,81 | 1,81 | 22,92 | **<0,0001** |
| Error | 28 | 2,21 | 0,08 | | |

It is important to underline that, in the absence of the group treated individually with PEA 5 mg/kg, the synergy test was conducted by comparing the group co-treated with PEA5 and Dicl3 with the groups treated individually with Dicl3 mg/kg and PEA10 mg/kg. Although the group treated individually with PEA at a double dosage compared to that treated with the PEA-NSAID combination, the analysis surprisingly confirmed the statistically significant superiority of the analgesic effect of the combination.

Similar observations emerged when verifying whether PEA-OXA exerted a synergistic effect on Diclofenac. Also in this case, the group co-treated with PEA-OXA (5 mg/kg) + Diclofenac (3 mg/kg) was compared (as well as with the group treated with Diclofenac alone, at the same dosage) with the group treated with PEA-OXA at double dosage. Surprisingly, the 2x2 ANOVA analysis however indicated that the effect of the association in reducing mechanical hyperalgesia is not additive but synergistic, i.e. significantly greater than the sum of the effects of the single treatments (p = 0.003, Table 9) .

Table 9 - Table resulting from the 2x2 ANOVA factorial analysis; the effect of the combined treatment (PEA-OXA5*dicl3) is significantly higher than the sum of the effects of the individual treatments, as highlighted by the probability value shown in bold in the last column of the table; the analysis confirms the synergistic effect exerted by combining PEA-OXA (5 mg/kg) with diclofenac

| Source | DF | Sum of Squares | Mean Squares | F Value | Pr > F |
|---|---|---|---|---|---|
| PEA-OXA10 | 1 | 4,41 | 4,41 | 49,08 | <0,0001 |
| dicl3 | 1 | 3,49 | 3,49 | 38,79 | <0,0001 |
| PEA-OXA5*dicl3 | 1 | 0,93 | 0,93 | 10,34 | **0,0033** |
| Error | 28 | 2,52 | 0,09 | | |

In light of the results set forth above, the inflammatory pain which can be treated according to the present invention is preferably selected from:

- Pain resulting from tissue injuries;
- Post-operative pain
- Toothache;
- Pain and inflammation of oral cavity and throat;
- Muscular and rheumatic pain;
- Menstrual cramps (dysmenorrhea);
- Inflammatory pain associated with capsulitis and bursitis;
- Inflammatory pain associated with tendonitis and tenosynovitis;
- Inflammatory pain associated with osteoarthritis;
- Inflammatory pain associated with periarthritis;
- Inflammatory pain associated with rheumatoid arthritis;
- Inflammatory pain associated with ankylosing spondylitis;
- Inflammatory pain associated with acute gout.

The invention will now be further described through the following formulation examples.

**Formulation examples**

um-PEA = Ultra-micronized palmitoylethanolamide
m-PEA = Micronized palmitoylethanolamide
non-m PEA = non-micronized palmitoylethanolamide
PEA-OXA = 2-pentadecyl-2-oxazoline

**Example 1 - Soft capsule for human use**

| | |
|---|---|
| Bovine gelatin | 155 mg |
| um-PEA | 50 mg |
| Diclofenac | 15 mg |
| Glycerol | 77.6 mg |
| Sunflower oil | 14 mg |
| Medium chain triglycerides | 80 mg |
| Vegetable fat | 90 mg |

(continued)

| | |
|---|---|
| Soy lecithin | 34 mg |
| Water | 11.6 mg |
| Glyceryl stearate | 12 mg |

**Example 2 - Soft capsule for human use**

| | |
|---|---|
| Bovine gelatin | 155 mg |
| um-PEA | 30 mg |
| Ketoprofen | 10 mg |
| Glycerol | 77.6 mg |
| Sunflower oil | 124 mg |
| Medium chain triglycerides | 100 mg |
| Vegetable fat | 100 mg |
| Soy lecithin | 34 mg |
| Water | 11.6 mg |
| Glyceryl stearate | 12 mg |

**Example 3 - Hard capsule for human use**

Acid-resistant vegetable gelatin capsule, "0" format

| | |
|---|---|
| um-PEA | 300 mg |
| Ketoprofen | 30 mg |
| Corn dextrin | 100 mg |
| Silicon dioxide | 10 mg |

**Example 4 - Gastro-resistant tablet**

| | |
|---|---|
| m-PEA | 500 mg |
| um-PEA | 100 mg |
| Diclofenac | 30 mg |
| Dextrose | 52 mg |
| Rice starch | 120 mg |
| Microcrystalline cellulose | 120 mg |
| Polysorbate 80, plant origin | 8 mg |
| Silicon dioxide | 10 mg |
| Gastro-resistant coating | 40 mg |

**Example 5 - Orosoluble granules**

| | |
|---|---|
| m-PEA | 300 mg |
| um-PEA | 300 mg |
| Ibuprofen | 100 mg |
| Sorbitol | 180 mg |

(continued)

| | |
|---|---|
| Fructose | 180 mg |
| Polysorbate | 8010 mg |
| PVP K30 | 10 mg |
| Sucrose Palmitate | 20 mg |

## Example 6 - Effervescent tablet

| | |
|---|---|
| m-PEA | 400 mg |
| um-PEA | 10 mg |
| Meloxicam | 3 mg |
| Corn dextrin | 500 mg |
| Potassium bicarbonate | 343 mg |
| Potassium carbonate | 108 mg |
| Anhydrous citric acid | 384 mg |
| Fructose | 130 mg |
| Polysorbate 80 | 15 mg |
| Lemon flavoring | 10 mg |

## Example 7 - Pediatric chewable tablet

| | |
|---|---|
| um-PEA | 300 mg |
| Ibuprofen | 100 mg |
| Sorbitol | 81.5 mg |
| Fructose | 220 mg |
| Croscarmellose | 50 mg |
| Citric acid | 22.4 mg |
| Flavoring | 6 mg |
| Magnesium stearate | 9 mg |
| Silicon dioxide | 9 mg |
| Sucrose palmitate | 6 mg |
| Dibasic calcium phosphate dihydrate | 94.1 mg |
| Microcrystalline cellulose | 100 mg |

## Example 8 - Pediatric suspension, 100 ml multi-dose bottle

| | |
|---|---|
| um-PEA | 4.0 g |
| Ibuprofen | 1.0 g |
| Sucrose | 30.0 g |
| Sodium carboxymethyl cellulose | 0.65 g |
| Microcrystalline cellulose | 1.35 g |
| Polysorbate 80 | 0.1 g |
| Potassium Sorbate | 0.1 g |
| Benzoic Acid | 0.075 g |
| Citric acid | 0.15 g |
| Flavoring | 0.1 g |

(continued)

Water q.s. to 100 ml

**Example 9 - Orosoluble granules**

| | |
|---|---|
| m-PEA | 300 mg |
| um-PEA | 300 mg |
| Ketoprofen | 25 mg |
| Sorbitol | 180 mg |
| Fructose | 180 mg |
| Polysorbate 80 | 10 mg |
| PVP K30 | 10 mg |
| Sucrose Palmitate | 20 mg |

**Example 10 - 2.0 g Suppositories**

| | |
|---|---|
| Suppository base (C10-18 Triglycerides-Polysorbate 65) | 1695 mg |
| non-mPEA | 200 mg |
| um-PEA | 100 mg |
| Diclofenac | 30 mg |

**Example 11 - Veterinary quadrisect tablet**

| | |
|---|---|
| m-PEA | 600 mg |
| um-PEA | 10 mg |
| Meloxicam | 3 mg |
| Palatability enhancer F20729 | 85 mg |
| Microcrystalline cellulose | 140 mg |
| Croscarmellose | 54 mg |
| Glyceryl Dibehenate | 90 mg |
| Hydroxypropyl cellulose | 20 mg |
| Polysorbate 80 | 5 mg |
| Magnesium Stearate | 6 mg |

**Example 12 - Gastro-resistant delayed-release tablet**

| | |
|---|---|
| um-PEA | 600 mg |
| Ketoprofen | 30 mg |
| Dextrose | 52 mg |
| Glyceryl dibehenate | 150 mg |
| Microcrystalline cellulose | 100 mg |
| Polysorbate 80, plant origin | 8 mg |
| Silicon dioxide | 10 mg |

(continued)

| | |
|---|---|
| Gastro-resistant coating | 40 mg |

## Example 13 - Combined formula

### Blister A - PEA tablets

| | |
|---|---|
| m-PEA | 600 mg |
| um-PEA | 10 mg |
| Dextrose | 52 mg |
| Rice starch | 120 mg |
| Microcrystalline cellulose | 120 mg |
| Polysorbate 80, plant origin | 8 mg |
| Silicon dioxide | 10 mg |

### Blister B - Ibuprofen tablets

| | |
|---|---|
| Ibuprofen | 300 mg |
| Dibasic calcium phosphate dihydrate | 140 mg |
| Magnesium stearate | 15 mg |
| Glyceryl dibehenate | 80 mg |
| Sorbitol | 100 mg |
| Gastro-resistant coating | 60 mg |

## Example 14 - Soft capsule for human use

| | |
|---|---|
| Bovine gelatin | 237 mg |
| um-PEA | 150 mg |
| Ketoprofen | 15 mg |
| Glycerol | 129 mg |
| Sunflower oil | 366 mg |
| Medium chain triglycerides | 51.5 mg |
| Vegetable fat | 50.8 mg |
| Soy lecithin | 30.0 mg |
| Water | 18.8 mg |
| Glyceryl stearate | 8.8 mg |

## Example 15 - Single-dose skin emulsion

| | |
|---|---|
| um-PEA | 100 mg |
| Diclofenac | 30 mg |
| Ethoxydiglycol | 300 mg |
| Sunflower lecithin | 30 mg |

(continued)

| Caprylic capric triglycerides | 150 mg |
| Glyceryl stearate | 30 mg |
| PEG100 Stearate | 30 mg |

Occlusive adhesive polymer dressing 10x10 cm

**Example 16 - Multi-dose injectable suspension 30 ml**

| um-PEA | 150 mg |
| Meloxicam | 45 mg |
| Polysorbate 80 | 600 mg |

Ethoxydiglycol q.s. to 30 ml

**Example 17 - Single-dose injectable solution**

| non-mPEA | 10 mg |
| Diclofenac | 3 mg |
| Soy lipids | 200 mg |
| Egg lecithin | 24 mg |
| Glycerol | 100 mg |

Water for injectables q.s. to 2 ml

**Example 18 - Hard capsule for human use**

| Acid-resistant vegetable gelatin capsule,"0" format | |
| um-PEA | 100 mg |
| PEA-OXA | 200 mg |
| Ketoprofen | 30 mg |
| Corn dextrin | 100 mg |
| Silicon dioxide | 10 mg |

**Example 19 - Gastro-resistant delayed-release tablet**

| PEA-OXA | 300 mg |
| um-PEA | 300 mg |
| Ibuprofen | 100 mg |
| Dextrose | 52 mg |
| Glyceryl dibehenate | 150 mg |
| Microcrystalline cellulose | 100 mg |
| Polysorbate 80, plant origin | 8 mg |
| Silicon dioxide | 10 mg |

(continued)

| | |
|---|---|
| Gastro-resistant coating | 40 mg |

## Example 20 - Soft capsule for human use

| | |
|---|---|
| Bovine gelatin | 237 mg |
| um-PEA | 100 mg |
| PEA-OXA | 50 mg |
| Diclofenac | 15 mg |
| Glycerol | 129 mg |
| Sunflower oil | 400 mg |
| Vegetable fat | 50.8 mg |
| Soy lecithin | 30.0 mg |
| Water | 18.8 mg |
| Glyceryl stearate | 8.8 mg |

## Example 21 - Hard capsule for human use

| | |
|---|---|
| Acid-fast vegetable gelatin capsule si"0" | |
| PEA-OXA | 200 mg |
| Diclofenac | 30 mg |
| Corn Dextrin | 100 mg |
| Magnesium Stearate | 8 mg |
| Silicon Dioxide | 10 mg |

## Example 22 - Gastro-resistant tablet

| | |
|---|---|
| PEA-OXA | 400 mg |
| Diclofenac | 30 mg |
| Dextrose | 52 mg |
| Rice starch | 80 mg |

| | |
|---|---|
| Microcrystalline cellulose | 120 mg |
| Polysorbate 80 vegetable origin | 8 mg |
| Silicon Dioxide | 10 mg |
| Magnesium Stearate | 8 mg |
| Gastro-resistant coating | 35 mg |

## Example 23 - Oral soluble granules

| | |
|---|---|
| PEA-OXA | 300 mg |
| Diclofenac | 25 mg |
| Sorbitol | 180 mg |

(continued)

| | |
|---|---|
| Glyceryl Palmitostearate | 40 mg |
| Fructose | 380 mg |
| Kolliphor ELP | 50 mg |
| PVP K30 | 10 mg |
| Polysorbate 80 | 10 mg |
| Sucrose Palmitate | 20 mg |
| Silicon Dioxide | 25 mg |
| Citric Acid | 50 mg |
| Lemon flavor | 10 mg. |

**Claims**

1. Palmitoylethanolamide for use in the treatment of inflammatory pain, in particular non-neuropathic inflammatory pain, wherein the palmitoylethanolamide is administered as needed in association or combination with a non-steroidal anti-inflammatory drug, wherein said administration is separate, combined, or simultaneous.

2. Palmitoylethanolamide for use according to claim 1, wherein the palmitoylethanolamide is in non-micronized form, having a particle size distribution, defined as a percentage by volume and measured by the laser light scattering method, represented by a distribution curve having the mode above 10 microns, preferably above 20 microns.

3. Palmitoylethanolamide for use according to claim 1, wherein the palmitoylethanolamide is in micronized form, having a particle size distribution, defined as a percentage by volume and measured by the laser light scattering method, represented by a distribution curve having the mode between 6 microns and 10 microns.

4. Palmitoylethanolamide for use according to claim 1, wherein the palmitoylethanolamide is in ultra-micronized form having a particle size distribution, defined as a percentage by volume and measured by the laser light scattering method, represented by a distribution curve having the mode below 6 microns and above 0.5 microns.

5. Palmitoylethanolamide for use according to claim 4, having a particle size distribution, defined as a percentage by volume and measured by the laser light scattering method, measured with a Malvern Mastersizer 3000 instrument with Fraunhofer calculation algorithm, wherein at least 90% by volume, preferably at least 95% by volume, of particles has a particle size less than 6 microns.

6. Palmitoylethanolamide for use according to claim 4, wherein palmitoylethanolamide has a particle size distribution, defined as a percentage by volume and measured by the laser light scattering method, measured with a Malvern Mastersizer 3000 instrument with Fraunhofer calculation algorithm, having a mode between 2 and 4 microns and having 100% by volume of particles less than 10 microns and at least 60% by volume of particles less than 3 microns.

7. Palmitoylethanolamide for use according to any one of claims 1 to 6, wherein PEA and NSAID are administered in a PEA/NSAID weight ratio between 20:1 and 1:1, preferably between 12:1 and 5:1.

8. Palmitoylethanolamide for use according to claim 7, wherein, when PEA is in ultra-micronized form, the PEA/NSAID weight ratio will preferably be between 11:1 and 3:1, more preferably between 10:1 and 5:1, and when PEA is in micronized or non-micronized form, the PEA/FANS weight ratio will preferably be between 20:1 and 5:1, more preferably between 18:1 and 10:1.

9. Palmitoylethanolamide for use according to any one of claims 1 to 8, wherein the overall daily dose of PEA administered to a subject is between 200 and 2000 mg/day, preferably between 300 and 1500 mg/day, or between 400 and 1200 mg/day.

10. Palmitoylethanolamide for use according to any one of claims 1 to 9, wherein palmitoylethanolamide and NSAID are contained in pharmaceutical or veterinary formulations and are formulated in dosage forms for oral, buccal, parenteral, rectal, topical or transdermal administration.

11. Palmitoylethanolamide for use according to any one of claims 1 to 9, wherein the palmitoylethanolamide is contained in dietary compositions, food supplements, complementary feed, or foods for special medical purposes (FSMP).

12. Palmitoylethanolamide for use according to any one of claims 1 to 11, wherein NSAID is selected from: Salicylates, such as acetylsalicylic acid; Acetic acid derivatives and the like, such as indomethacin, diclofenac, ketorolac, aceclofenac; Propionic acid derivatives, such as ibuprofen, ketoprofen, and naproxen; Oxicam derivatives, such as piroxicam and meloxicam; Phenamates such as mefenamic acid; Coxib or COX-2 inhibitors, such as celocoxib, etoricoxib, and parecoxib; Nimesulide; Normiflumate/niflumic acid.

13. Palmitoylethanolamide for use according to any one of claims 1 to 12, further comprising the administration of 2-pentadecyl-2-oxazoline.

14. Palmitoylethanolamide for use according to any one of claims 1 to 13, wherein the non-neuropathic inflammatory pain is a pain selected from:

- Pain resulting from tissue injuries;
- Post-operative pain;
- Toothache;
- Pain and inflammation of oral cavity and throat;
- Muscular and rheumatic pain;
- Menstrual cramps (dysmenorrhea);
- Inflammatory pain associated with capsulitis and bursitis;
- Inflammatory pain associated with tendonitis and tenosynovitis;
- Inflammatory pain associated with osteoarthritis;
- Inflammatory pain associated with periarthritis;
- Inflammatory pain associated with rheumatoid arthritis;
- Inflammatory pain associated with ankylosing spondylitis;
- Inflammatory pain associated with acute gout.

15. A composition comprising or consisting of a mixture of palmitoylethanolamide, preferably ultra-micronized palmitoylethanolamide, a non-steroidal anti-inflammatory drug, pharmaceutically acceptable excipients, and optionally 2-pentadecyl-2-oxazoline, wherein the PEA/NSAID weight ratio is between 20:1 and 1:1, preferably between 12:1 and 5:1, wherein PEA is contained in an amount between 200 and 2000 mg and wherein NSAID is preferably selected from diclofenac, meloxicam, ibuprofen, and ketoprofen.

16. 2-Pentadecyl-2-oxazoline for use in the treatment of inflammatory pain, in particular non-neuropathic inflammatory pain, where 2-pentadecyl-2-oxazoline is administered in association or combination with a non-steroidal anti-inflammatory drug, wherein said administration is separate, joint or simultaneous.

17. 2-Pentadecyl-2-oxazoline for use according to claim 16, wherein the nonsteroidal anti-inflammatory drug is diclofenac.

18. 2-Pentadecyl-2-oxazoline for use according to claim 17, wherein the weight ratio 2-pentadecyl-2-oxazoline/diclofenac is 5:3 or greater.

19. 2-Pentadecyl-2-oxazoline for use according to any one of claims 16 to 18, wherein 2-pentadecyl-2-oxazoline is administered at a dose of between 100 mg and 1000 mg per day.

20. A composition comprising or consisting of a mixture of 2-pentadecyl-2-oxazoline, a non-steroidal anti-inflammatory drug, preferably diclofenac, and pharmaceutically acceptable excipients, in which the 2-pentadecyl-2-oxazoline is preferably contained in an amount of between 100 mg and 1000 mg and in which the weight ratio 2-pentadecyl-2-oxazoline/diclofenac is preferably 5:3 or greater.

Figure 1

Figure 2

Figure 3

Figure 4

Figure 5

Figure 6

Figure 7

Figure 8

Figure 9

Figure 10

Europäisches
Patentamt

European
Patent Office

Office européen
des brevets

## EUROPEAN SEARCH REPORT

**Application Number**

EP 25 15 6611

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/031749 A1 (SHILPA MEDICARE LTD [IN]) 9 March 2023 (2023-03-09) <br> * paragraph [0019] - paragraph [0020] * <br> * paragraph [0028] - paragraph [0042] * <br> * claim 10 * <br> - - - - - | 1-15 | INV. <br> A61K31/164 <br> A61K31/196 <br> A61K9/00 <br> A61K9/107 <br> A61K9/48 <br> A61K31/5415 <br> A61P29/00 <br> A61K31/192 |
| X | WO 2013/063263 A1 (LYCUS LLC [US]; SESHA RAMESH [US]) 2 May 2013 (2013-05-02) <br> * paragraph [0022] - paragraph [0024] * <br> * paragraph [0037] * <br> * paragraph [0070] * <br> * paragraphs [00119] - [00120] * <br> * claims 1,12-14,18, 29-32 * <br> * paragraph [0130] * <br> - - - - - | 1-15 | |
| X | WO 2016/183134 A1 (CUTTING EDGE MEDICAL SOLUTIONS LLC [US]) 17 November 2016 (2016-11-17) <br> * claims 1, 130, 131 * <br> * page 41, last paragraph - page 42, paragraph 2 * <br> - - - - - | 1,7,10, 12,15 | |
| X | CHIRCHIGLIA DOMENICO ET AL: "Effects of Add-On Ultramicronized N-Palmitol Ethanol Amide in Patients Suffering of Migraine With Aura: A Pilot Study", FRONTIERS IN NEUROLOGY, vol. 9, 17 August 2018 (2018-08-17), XP093193478, ISSN: 1664-2295, DOI: 10.3389/fneur.2018.00674 | 1,4-10, 12,14,15 | **TECHNICAL FIELDS SEARCHED (IPC)** <br> A61K <br> A61P |
| Y | * the whole document * <br> * tables 1-5 * <br> * page 2, right-hand column, paragraph 2-4 * <br> - - - - - <br> -/-- | 1-15 | |

The present search report has been drawn up for all claims

| Place of search | Date of completion of the search | Examiner |
|---|---|---|
| Munich | 29 August 2025 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 15 6611

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/290609 A1 (DELLA VALLE FRANCESCO [IT] ET AL) 26 September 2019 (2019-09-26) <br> * paragraph [0002] * <br> * paragraph [0017] - paragraph [0023] * <br> * paragraph [0031] * <br> * claims 1,2,9,10 * <br> ----- | 1-15 | |
| Y | MOLCHANOVA A. IU. ET AL: "ANTINOCYCEPTIVE EFFECT OF A SYSTEMIC ADMINISTRATION OF PALMITOYLETHANOLAMIDE, STEAROYLETHANOLAMIDE AND DYNCLOFENAC IN RATS WITH EXPERIMENTAL NEUROGENIC PAIN SYNDROME", <br> PROCEEDINGS OF THE NATIONAL ACADEMY OF SCIENCES OF BELARUS, MEDICAL SERIES , <br> vol. 15, no. 3 <br> 16 September 2018 (2018-09-16), pages 331-338, XP093193475, <br> ISSN: 1814-6023, DOI: 10.29235/1814-6023-2018-15-3-331- <br> Retrieved from the Internet: <br> URL:https://vestimed.belnauka.by/jour/article/viewFile/450/440 <br> * the whole document * <br> ----- | 1-15 | |
| A | DOMENICO BRITTI ET AL: "A novel composite formulation of palmitoylethanolamide and quercetin decreases inflammation and relieves pain in inflammatory and osteoarthritic pain models", <br> BMC VETERINARY RESEARCH, <br> vol. 13, no. 1, 2 August 2017 (2017-08-02) , XP055544779, <br> DOI: 10.1186/s12917-017-1151-z <br> * the whole document * <br> ----- | 1-15 | **TECHNICAL FIELDS SEARCHED (IPC)** |

The present search report has been drawn up for all claims

| Place of search | Date of completion of the search | Examiner |
|---|---|---|
| Munich | 29 August 2025 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 6611

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2025

| Patent document cited in search report | Publication date | Patent family member(s) | Publication date |
|---|---|---|---|
| WO 2023031749 A1 | 09-03-2023 | NONE | |
| WO 2013063263 A1 | 02-05-2013 | WO 2013063263 A1 | 02-05-2013 |
| | | WO 2013063289 A1 | 02-05-2013 |
| WO 2016183134 A1 | 17-11-2016 | NONE | |
| US 2019290609 A1 | 26-09-2019 | US 2019290609 A1 | 26-09-2019 |
| | | US 2023285349 A1 | 14-09-2023 |

EPO FORM P0459

For more details about this annex : see Official Journal of the European Patent Office, No. 12/82

## REFERENCES CITED IN THE DESCRIPTION

**Non-patent literature cited in the description**

- **SAMAL et al.** *J Maxillofac Oral Surg.*, March 2021, vol. 20 (1), 63-69
- **RINGSTEN et al.** *Cochrane Database Syst Rev.*, 11 December 2023, vol. 12 (12), CD015087
- **BINDU et al.** *Biochem Pharmacol.*, October 2020, vol. 180, 114147
- **LASCELLES et al.** *Vet Ther.*, 2005, vol. 6 (3), 237-51
- **WERNHAM et al.** *Aust Vet J.*, March 2023, vol. 101 (3), 90-98
- **NESTMANN**. *Food Sci Nutr.*, 15 June 2016, vol. 5 (2), 292-309
- Remington's Pharmaceutical Sciences Handbook. Mack Pub. Co., 1985
- Remington, The Science and Practice of Pharmacy. 2012
- **LEIGHTON GE et al.** kappa-Opioid agonists produce antinociception after i.v. and i.c.v. but not intrathecal administration in the rat. *Br J Pharmacol.*, 1988, vol. 93, 553-60
- **SLINKER BK**. The statistics of synergism.. *J Mol Cell Cardiol.*, April 1998, vol. 30 (4), 723-31
- **IMPELLIZZERI D et al.** *J Pharmacol Exp Ther*, 2011, vol. 33, 859-869
- **BUTLER et al.** *Pain*, 1992, vol. 48, 73-81
- **LEIGHTON et al.** *Br J Pharmacol*, 1988, vol. 93, 553-560
- **FERNANDES et al.** *Arthritis Res Ther.*, 11 January 2016, vol. 18, 7